Europäisches Patentamt

European Patent Office

Office européen des brevets

(11) Publication number: **0 055 056**
**A1**

# EUROPEAN PATENT APPLICATION

(21) Application number: 81305839.3

(22) Date of filing: 11.12.81

(51) Int. Cl.³: **D 01 F 2/08, A 61 L 15/00,**
**C 08 L 1/24**

(30) Priority: 22.12.80 US 219134

(71) Applicant: **Rohm and Haas Company, Independence Mall West, Philadelphia, Pennsylvania 19105 (US)**

(43) Date of publication of application: 30.06.82
Bulletin 82/26

(72) Inventor: **Smith, Frederick Ray, Route 1 Box 103-G, Toms Brook Virginia 22660 (US)**
Inventor: **Witiak, David, 500 Stony Hill Road, Yardley Pennsylvania 19067 (US)**
Inventor: **Toy, Walter Weiyoung, 849 Concord Place, Lansdale Pennsylvania 19446 (US)**

(74) Representative: **Angell, David Whilton et al, Rohm and Haas Company Patent Department Chesterfield House Barter Street, London WC1A 2TP (GB)**

(84) Designated Contracting States: **AT BE CH DE FR GB IT LI NL SE**

(54) **Alloy rayon fibers of high fluid-holding capacity, method of producing them and manufactured articles comprising them.**

(57) Alloy rayon fibers of high fluid holding capacity are obtained by incorporating into the viscose used to prepare the fibers by spinning into an acid spinning bath an acrylic copolymer comprising acrylic or methacrylic acid and a hydroxyalkyl acrylate or methacrylate or a styrenic compound as comonomer, and at least partially neutralizing the carboxy groups present in the resulting fiber.

EP 0 055 056 A1

1

## ALLOY RAYON FIBERS OF HIGH FLUID-HOLDING CAPACITY, METHOD OF PRODUCING THEM AND MANUFACTURED ARTICLES COMPRISING THEM

The present invention relates to alloy rayon fibers having high fluid-holding capacity, to articles comprising such fibers, and to a method of preparing the fibers.

Alloy rayon fibers are known and comprise a homogeneous blend of regenerated cellulose and a substance capable of increasing the fluid-holding capacity of the fibers. Such fibers are to be distinguished over regenerated cellulose fibers that are merely coated with the substance. Such alloy rayon fibers are usually obtained from a uniform mixture of an aqueous alkaline cellulose xanthate solution and the alloying component. In this specification and claims "fluid-holding capacity" refers to the total liquid taken up by a mass of such alloy fibers, including the liquid held within the interstices of a mass of the fibers and the liquid absorbed and adsorbed by the fibers.

In making such high fluid-holding alloy rayon fibers it is known to use various acrylic polymers as the alloying component, the acrylic polymer being incorporated into the regenerated cellulose by addition to the viscose used to form the regenerated cellulose fibers and spinning the resulting mixture in the conventional way. Representative prior patents are U.S. Patents 3,844,287 (now Reissue 30,029), 4,066,584 and 4,104,214. Such prior proposals, however, are generally characterized by undesirable losses of the acrylic polymer to and interaction of the acrylic polymer with the aqueous acidic spin bath, usually an aqueous solution of $H_2SO_4$, and $Na_2SO_4$, into which the mixture of the acrylic polymer and viscose are spun to form the alloy rayon fiber.

Such acrylic polymers lost from the cellulosic matrix during filament formation produce flocculates and/or agglomerates with spinning by-product materials. In turn, these become tacky, troublesome deposits in the spin bath system. The deposits are difficult to remove from the system and special procedures are required to deal with them.

In accordance with this invention it has been discovered that such problems can be minimised by using as the acrylic polymer a copolymer of (a) acrylic acid or methacrylic acid or a mixture of the two and (b) at least one comonomer selected from hydroxyalkyl $(C_2-C_6)$ esters of said acids and styrenic monomers.

By "styrenic monomer" as used herein is meant styrene or any vinyl aromatic monomer having properties substantially equivalent to styrene when used as a comonomer in preparing the copolymers herein. "Styrenic monomers" thus include styrene and substituted styrenes such as the various alkyl- or halogen-substituted styrenes including the ortho, meta and para isomers thereof, for example: vinyl toluene, ethylstyrene, p-chlorostyrene, 2,4-dichlorostyrene, α-methyl styrene, and the like.

In particular, it has been found that by using such an acrylic polymer homogeneously mixed and/or uniformly dispersed in desired amounts with viscose, the resulting mixture or solution is spinnable into rayon by well known methods so as to form a high-fluid-holding regenerated cellulosic alloy fiber and that the spin bath used in making the alloy fiber will be devoid of or contain only minimal amounts of tacky contaminant, and that there are virtually no losses of acrylic polymer during regeneration of the viscose/acrylic polymer spun from the spinning jets, and during subsequent processing of the regenerated cellulosic fibers.

The monomer ratio (a:b) in the copolymer used in accordance with this invention ranges from 95:5 to 50:50 by weight, preferably from 90:10 to 60:40. Choice of comonomer (b) for copolymerization with the acid monomer (a), the monomer ratio a:b and copolymer molecular weight will be guided by several considerations. These include: the viscosity and solubility of the copolymer in aqueous solution at different pH values and polymer concentrations, copolymer stability, degree of enhancement of fluid holding capacity, avoidance of formation of sticky deposits during alloy fiber formation, and the relative costs of the monomers and copolymer

manufacture. Thus, for a given molecular weight, the solubility of the copolymer will increase as the pH is raised and will decrease as the proportion of comonomer (b) increases. Solubility will also decrease as the carbon content of comonomer (b) increases. For example, in a copolymer of a given acid monomer, molecular weight and monomer ratio, hydroxypropyl methacrylate and hydroxybutyl methacrylate will provide lower solubility than will the corresponding hydroxyethyl methacrylate, and styrene will introduce even lower solubility. Such lower solubilities are desirable under the acidic conditions of the spin bath for coagulating and regenerating cellulosic fibers, in order to avoid or minimize formation of sticky deposits of copolymer not incorporated into the fiber. However, the same copolymer must be sufficiently soluble under the alkaline conditions of the viscose for good admixture with the viscose. The rayon alloy fiber chemist fully understands and appreciates the foregoing considerations and can readily vary the aforementioned parameters to optimize the benefits of the invention, in light of the inventive aspects described herein and the skill of the art.

Among the specific acrylic polymers found useful in this invention are copolymers of acrylic or methacrylic acid with one or more of hydroxyethyl acrylate ("HEA"), hydroxyethyl methacrylate ("HEMA"), hydroxypropyl acrylate ("HPA") and styrene. When the esterifying alcohols are isomeric mixtures, the ester groups will be a mixture of the various isomers --- e.g., hydroxypropyl acrylate will comprise a mixture of the 2-hydroxypropyl and 3-hydroxypropyl esters. These copolymers are usually prepared in an aqueous solution containing about 5 to 20 percent solids (polymer), and may be in acid form or in partially or completely neutralized form.

The copolymers may contain minor proportions -- e.g., up to about 25 wt. percent -- of other monomers, in addition to monomers (a) and (b), provided the type and amount are such as not to detract materially from the properties required for use in this invention. Appropriate other monomers include any monomers which are monofunctional to monomers (a) and (b) and therefore will not introduce cross-linking. Among acceptable other monomers are esters of acrylic and methacrylic acids, such as the $C_1 - C_{18}$ alkyl esters thereof, vinyl esters such as vinyl acetate, and vinyl halides such as vinyl chloride, and the like.

The copolymers are prepared in aqueous dispersion by conventional, free radical solution polymerization techniques to provide products having molecular weights (weight average) in the range of about 10,000 to about 500,000, preferably about 300,000 to about 400,000. Molecular weights substantially under 10,000 will not provide sufficient fluid-holding capacity, and molecular weights over 500,000 render the copolymer solutions too viscous for efficient and economical handling and incorporation into the viscose.

It has been observed that the copolymers used in this invention are initially water-soluble or marginally water-soluble but over a relatively short period of time became irreversibly gelatinous. This unstable condition shortens the useful life of the copolymers and as a practical matter will make their use as alloying components of rayon uneconomic. Therefore it is preferred to use copolymers which have been at least partially neutralized simultaneously with or soon after formation in solution. Not only is the tendency to gellation eliminated or minimized thereby, but the marginally water soluble copolymers are solubilized and the viscosity of the solutions is maintained in a manageable range.

The copolymers should be preneutralized at least about 25 percent, i.e., at least about 25 percent of the carboxyl groups of the copolymer should be converted to water-solubilizing salt form, preferably by treatment of the copolymers with an alkali metal hydroxide (e.g. NaOH or KOH) or ammonium hydroxide. Generally, up to about 75 percent neutralization is sufficient; higher degrees of neutralization tend to unduly increase the viscosity of the solutions. Best results have been observed for about 50% to 75% neutralization with copolymers of the preferred molecular weight range, for solutions containing 5 to 20 wt. % of the neutralized copolymer. Thus, the degree of neutralization will generally be in inverse proportion to molecular weight for solutions of the same polymer solids, since both properties contribute to viscosity of the copolymer solutions.

The above described technique for the stabilization of such polymers by controlled neutralization is the subject of our copending European application No          filed simultaneously herewith. and claiming priority from United States Serial No. 218 909

In the present invention the acrylic copolymer may be added as an aqueous dispersion to the viscose prior to spinning. The mixture is then spun into fiber form in an acid spin bath in a conventional manner as described, for example, in US Patents 4,066,584 and 4,104,214. Following spinning the fibers are recovered from the bath, washed to remove adhering acid, neutralized to convert or reconvert carboxyl groups in the copolymer to salt form, and then dried.

Preferably, the alloy rayon fibers of the invention will contain about 5 to 40%, based on the weight of cellulose ("boc"), preferably 10 to 30%, of the copolymer, and the alloy fiber will be neutralized sufficiently to provide fluid-holding capacity of at least 5.5 cc/gram, as measured by the Syngyna test (described hereinafter). The fiber preferably is neutralized to the required extent, e.g. at least 45%, by treatment with an alkali metal hydroxide, such as NaOH, or ammonium hydroxide, with the result that substantially all or a portion of the carboxyl groups of the copolymer are converted to the corresponding salts.

The fibers may also be treated in known manner to provide a lubricant finish of, for example, a water-soluble polyoxyethylene sorbitan mono-laurate, or similar nonionic polyoxyethylene sorbitan monoester of higher fatty acid or other nonionic material. Other types of finishes known in the art may be used. The type and amount of fiber finish and the degree of conversion of carboxyl groups to carboxylate salt groups are selected such that the wet fibers have considerable resistance to compaction and tend to separate from each other after they have been squeezed together (to express excess water) under pressure and then released. Thus, when the fibers are dried they will show little tendency to adhere to neighboring fibers, and the product fibers after "opening" will be substantially nonbonded. Specifically, the proportion of finish and the proportion of the carboxyl groups which are in salt form (as the sodium salt) should be such that when a mass of the fibers is wetted with two or more times its dry weight and then dried, without tension, in air at $25^{0}$C, the resulting fibers are substantially nonadherent. A desirable fiber pH (measured on a 1% slurry of these lubricated fibers in deionized water) is in the range of about 5.5 to 9.0, preferably about 6 to 8.5. Generally, the fibers are of about 1.5 to 6 denier and the staple fiber length is in the range of about 3/4 to 5 inches, (2-13 cms), such as about 1-1/2 inches (3.8 cms).

The fiber may be crimped, as described, for instance, in Merion et al. U.S. 2,517,694; Textile Research Journal Vol. 23 pp. 137-157 and Man Made Fibers by Moncrief (6th Edition, 1975, publ. by John Wiley & Sons) pp. 191-193.

The alloy fibers of the present invention are adapted for use in a variety of articles, such as surgical dressings, pads, and vaginal tampons, in which high fluid holding retention is an essential characteristic. In the manufacture of such articles, the alloy fibers may be used in the same manner and with the same equipment as employed with other commercial fibers, including regular rayon. They may be blended with other fibers which may or may not enhance the absorbent properties of the resulting articles.

Staple fibers with which the alloy fibers of the present invention may be blended include, for example, rayon, cotton, chemically modified rayon or cotton, cellulose acetate, nylon, polyester, acrylic, polyolefin, etc. Typically, a tampon is an elongated cylindrical mass of compressed fibers, which may be supplied within a tube which serves as an applicator; see U.S. Patents Nos. 2,024,218; 2,587,717; 3,005,456; 3,051,177.

The following examples are intended as further illustrations of the invention. All parts and percentages are by weight unless otherwise specified.

In the Examples the fluid holding capacity is measured by the following procedure: The fibers are carded into web form
and then separated into 2.5 gram portions each about 6 inches
(15 cms) long. Each sub web portion is then individually rolled
in the direction of its width to provide a roll six inches long
(15 cms) and a string is looped about the center thereof. Each
such roll is then folded on itself at the string loop and drawn
into a 1/2 inch diameter (1.27 cm) tube within which it is
compressed by a clamp and plunger, thus forming a tampon. The
resulting tampons are removed, and allowed to stand for a period
of about 30 minutes (during which the tampons recover to a bulk
density of about 0.4 g/cc). They are then evaluated for their
capacity to hold water by the Syngyna Method, essentially as
described by G.W. Rapp in a June 1958 publication of the
Department of Research, Loyola University, Chicago, Illinois,
titled "A Comparison of the Absorptive Efficiency of the Commercial
Catamenial Tampon." but modified in that the liquid supply means
was a syringe pump which supplied liquid to the tampon at a
fixed rate of 0.92 milliliters per minute. When the end of the
test is observed, the volume of liquid delivered is divided by
the known weight of the tampon and the quotient is recorded.

### EXAMPLES

Example I

Preparation of Acrylic acid/hydroxyethyl methacrylate
(80/20 wt. %) Copolymer.

Into a 5-liter 3-neck round bottom flask is charged 2960
ml of deionized (DI) water. The water is heated with a heating
mantle to 85-87°C while being stirred with a mechanical stirrer.
While this is being done, 416 g of acrylic acid (AA) and 104 g
of 2-hydroxyethyl methacrylate (HEMA) are charged into a one-
liter graduated separatory funnel. A 1 wt. % aqueous solution
of ammonium persulfate (APS) is also prepared. When the temperature of the water levels off at 85-87°C, 13 ml of the 1 wt. % APS
solution is added to the reactor. The monomer solution and a co-
feed catalyst of 52 ml of the 1 wt. % APS solution are then
added gradually and proportionally to the reactor over a period of about two hours. During the addition the temperature is maintained at 85-87°C and agitation is increased gradually to maintain good stirring.

After the additions are completed the polymer solution is held at 85-87°C for 30 minutes. At the end of the 30-minute time period, an additional catalyst system consisting of 0.4 g of tertiary butyl hydroperoxide diluted in 10 ml of deionized water and 0.3 g of sodium sulfoxylate formaldehyde dissolved in 10 ml of deionized water are added. Stirring is continued for 30 minutes. A neutralizer consisting of 232 g of 50% sodium hydroxide is added. Stirring is continued for about 30 minutes. The product obtained is a clear solution with a viscosity of about 7000 cps. at $20°C \pm 2°C$ (Brookfield Viscometer, 12 rpm spindle 3) and has a polymer solids content of 15.4% with about 50% of the acid groups neutralized.

Example II

The copolymer solution in water, prepared as described in Example I, a viscous liquid, containing about 15% of the copolymer of acrylic acid and hydroxyethyl methacrylate (HEMA), was injected into viscose so that the resulting solutions of viscose and copolymer at the spinning jet contained 15, 20 and 25 percent of copolymer (calculated as fully neutral salt), based on cellulose (boc) in the viscose. During blending, the mixture is subjected to mechanical shearing. The composition of the viscose (prior to blending) is 9.6 percent cellulose, 6.2 percent sodium hydroxide and 31 percent (based upon the weight of the cellulose) carbon disulfide. The viscose ballfall viscosity is 60 and its common salt test is 7.

The spin bath contained 7.6 percent $H_2SO_4$, 21 percent $Na_2SO_4$ and one percent $Zn SO_4$ at 55°C. The stretch bath contained 5 percent $H_2SO_4$ and 10 percent $Na_2SO_4$ and the stretch was 70 percent. The fiber was collected, cut to about 1 1/2 inch lengths, washed successively in hot water - 20 minutes, 0.5 percent NaOH - 10 minutes, water - 5 minutes for each of four washings, and finally washed in a solution containing 0.3 percent AHCO (R) 7596T solution. (AHCO is a trademark of ICI America for a series of polyoxyethylene adducts of sorbitan monoesters of higher fatty acids. AHCO 7596T is the lauric acid derivative.) A portion of the fiber produced from the solution containing 20 percent polymer (boc) was set aside in an acid form, that is, after the fibers were washed but before neutralization. This portion is identified as sample D. The fiber samples were dried, conditioned at 70°C and 65 percent relative humidity and tested, giving results as follows (Table I) wherein samples II-A, II-B and II-C are the fibers prepared from the viscose solutions containing 15, 20 and 25 percent, respectively, of the acrylic copolymer salt.

TABLE I

| | Polymer Percent (boc) | Syngyna Value cc/g | Water Retention Percent | Saline Retention Percent | Saline pH |
|---|---|---|---|---|---|
| Sample II-A | 15 | 5.45 | 154 | 135 | 6.7 |
| Sample II-B | 20 | 6.05 | 180 | 152 | 6.8 |
| Sample II-C | 25 | 5.67 | 195 | 159 | 6.6 |
| Control | Regular rayon | 3.8-4.2 | 110 | – | – |

Example III

The fiber sample portion D set aside in acid state, as described in Example II, was treated twice in 0.5 percent $H_2SO_4$ to assure substantially complete conversion of the contained polymer to the acid form. The sample was then washed with water to remove the $H_2SO_4$. Portions of the sample (except a control sample portion 1) were individually treated with dilute NaOH solution, varying the concentration to achieve different degrees of neutralization. The resulting fibers were washed, and a finish was applied as in Example II. The fibers were then dried. The control sample portion 1 (acid state) was similarly dried. Each sample portion was evaluated in the Syngyna test with results as shown in Table II.

0055056

10

## TABLE II

| Fiber Sample Portion D | 1 | 2 | 3 | 4 | 5 | 6 |
|---|---|---|---|---|---|---|
| Syngyna cc/g | 3.89 | 5.16 | 5.82 | 6.04 | 6.05 | 6.35 |
| Saline pH | 3.04 | 4.35 | 4.96 | 5.32 | 5.87 | 6.61 |
| Degree of neutralization | .05 | .36 | .56 | .66 | .79 | .92 |

This experiment shows the importance of substantial conversion of the fiber to the neutralized form, in this case the sodium salt, in that at least partial neutralization is required to increase the fluid holding capacity over that exhibited by alloy fiber containing essentially unneutralized acrylic polymer.

Example IV

Copolymers of AA/HEMA were prepared as in Example I by varying the monomer ratio to increase HEMA content. The absorbency values for the fibers made from viscose with these copolymers are presented in Table III. (Fiber Samples II-B, III-B, III-C and III-D).

Alternately, when HPMA, HEA and HPA were substituted for HEMA as the minor monomer of the copolymer and polymerized with AA, the below-listed copolymers were obtained. (Fiber Samples III-E to III-H).

Likewise, when methacrylic acid was substituted for acrylic acid in the synthesis procedure of Example I, the corresponding listed products were prepared. (Fiber samples III-I and III-J). Similarly, a copolymer of acrylic acid was prepared with styrene as the comonomer, spun into a fiber and evaluated for fluid holding capacity. (Fiber Sample K).

## TABLE III

| Fiber Sample | Major Monomers | Minor Monomers | Copolymer (monomer ratios) | Syngyna Values (Average Three) |
|---|---|---|---|---|
| II-B | Acrylic Acid (AA) | Hydroxyethyl Methacrylate (HEMA) | AA/HEMA 80/20 | 6.0 |
| III-B | Acrylic Acid (AA) | Hydroxyethyl Methacrylate (HEMA) | AA/HEMA 70/30 | 6.0 |
| III-C | Acrylic Acid (AA) | Hydroxyethyl Methacrylate (HEMA) | AA/HEMA 65/35 | 6.4 |
| III-D | Acrylic Acid (AA) | Hydroxyethyl Methacrylate (HEMA) | AA/HEMA 60/40 | 6.6 |
| III-E | Acrylic Acid (AA) | Hydroxypropyl Methacrylate (HPMA) | AA/HPMA 80/20 | 6.42 |
| III-F | Acrylic Acid (AA) | Hydroxypropyl Methacrylate (HPMA) | AA/HPMA 90/10 | 6.33 |
| III-G | Acrylic Acid (AA) | Hydroxyethyl acrylate (HEA) | AA/HEA 80/20 | 6.57 |
| III-H | Acrylic Acid (AA) | Hydroxypropyl acrylate (HPA) | AA/HPA 80/20 | 5.64 |
| III-I | Methacrylic Acid (MAA) | Hydroxyethyl Methacrylate (HEMA) | MAA/HEMA 80/20 | 5.75 |
| III-J | Methacrylic Acid (MAA) | Hydroxypropyl Methacrylate (HPMA) | MAA/HPMA 80/20 | 5.25 |
| III-K | Acrylic Acid (AA) | Styrene (STY) | AA/STY 85/15 | 6.2 |
| III-L | None | None | None | 3.8-4.2 |

Table III presents Syngyna test results for the foregoing alloy fibers of the invention (fiber samples III-B to III-K) as compared with alloy fiber sample II-B of Table II and a regular, non-alloyed fiber sample III-L as control. The alloy fibers were prepared substantially as described in Example II from a fluid containing viscose and containing 20 percent polymer (boc). It can be seen that the copolymer substantially increased the fluid holding capacity of the fibers.

Example V

The effect of the acidic spin bath on some of the polymers used in forming the alloy fibers of the invention was studied, as compared to polyacrylic acid as a control. In each case, the polymers were in 50 percent neutralized form (sodium salts). A small pocket was fashioned by folding filter paper. One or two grams of a polymer solution was placed in the pocket and the pocket was then suspended in a synthetic spin bath, containing 7 percent $H_2SO_4$ and 21 percent $Na_2SO_4$. After 30 minutes the pocket was removed and the contents examined and classified accordingly to appearance (stringy, gel or curd) and touch (stickiness). Results in Table IV.

### TABLE IV

#### PROPERTIES OF RESIDUE FROM POLYMER/SPIN BATH INTERACTION

| POLYMER | RATIO | APPEARANCE OF RESIDUE | TACTILE |
|---------|-------|----------------------|---------|
| AA/HEMA | 80/20 | gel, stringy | non sticky |
| AA/HEMA | 70/30 | gel, curd | non sticky |
| AA/HEMA | 65/35 | gel, curd | non sticky |
| AA/HEMA | 60/40 | gel, curd | non sticky |
| AA/HPMA | 80/20 | stringy | non sticky |
| AA/HPMA | 90/10 | stringy | sticky |
| AA/HEA | 80/20 | elastic gel | sticky |
| MAA/HPA | 80/20 | curd | sticky |
| MAA/HEMA | 80/20 | gel | sticky |
| MAA/HPMA | 80/20 | gel | sticky |
| AA/MAA | 50/50 | stringy | sticky |
| AA | – | stringy | extremely sticky |

CLAIMS

1    An alloy rayon fiber comprising a blend of regenerated cellulose and acrylic polymer effective to increase the fluid holding capacity of the regenerated cellulose, characterised in that the acrylic copolymer is an at least partially neutralized copolymer of a) acrylic or methacrylic acid or a mixture of the two, and b) at least one comonomer selected from $C_2$-$C_6$ hydroxyalkyl esters of acrylic or methacrylic acid and styrenic comonomers, in a monomer ratio a:b of from 95:5 to 50:50, said copolymer having a molecular weight in the range 10,000 to 500,000.

2    An alloy rayon fiber according to claim 1, characterised in that the monomer ratio in said copolymer is from 90:10 to 60:40.

3    An alloy rayon fiber according to claim 1 or 2, characterised in that the copolymer is present in an amount of from 5 to 40% by weight based on the regenerated cellulose.

4    An alloy rayon fiber according to any one of claims 1-3, characterised in that the copolymer is at least 45% neutralized.

5    An alloy rayon fiber according to any one of claims 1-4, characterised in that the copolymer is at least partially in alkali metal or ammonium salt form.

6    An alloy rayon fiber according to any one of claims 1-5, characterised in that fluid holding capacity of the fiber is at least 5 cc/g in the Syngyna test as hereinbefore defined.

7    An alloy of rayon fiber according to any one of claims 1-6, characterised in that component b) of the copolymer is hydroxyethyl or hydroxypropyl acrylate or methacrylate.

                                    producing
8.      A method of/an alloy rayon fiber which comprises spinning
a viscose into an aqueous acidic spinning bath, and recovering
the spun fiber, characterised in that there is incorporated into
the viscose a copolymer of a) acrylic or methacrylic acid, and b)
a $C_2$-$C_6$ hydroxyalkyl ester of acrylic or methacrylic acid, or a
styrenic comonomer in a monomer ratio a:b of from 95:5 to 50:50,
said copolymer having a molecular weight in the range 10,000 to
500,000 and in that after recovery from the bath the acid groups
in the spun fiber are at least partially neutralized.


9       A method according to claim 8, characterised in that
25-75% of the acid groups in the copolymer are neutralized prior
to incorporation of said copolymer into said viscose.


10      A method according to claim 8 or 9, characterised in that
the copolymer is added to the viscose in an amount of from 5-40%
by weight based on the weight of the cellulose.


11      A fluid absorbing article comprising a mass of alloy
rayon fibers as claimed in any one of claims 1-7.

## DOCUMENTS CONSIDERED TO BE RELEVANT

| Category | Citation of document with indication, where appropriate, of relevant passages | Relevant to claim |
|---|---|---|
| DA | US - A - 4 066 584 (T.C. ALLEN et al.) -- | |
| DA | US - A - 4 104 214 (A.W. MEIER-HOEFER) ---- | |

**CLASSIFICATION OF THE APPLICATION (Int. Cl. 3)**

D 01 F 2/08
A 61 L 15/00
C 08 L 1/24

**TECHNICAL FIELDS SEARCHED (Int.Cl. 3)**

D 01 F
A 61 L
C 08 L

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document

The present search report has been drawn up for all claims

| Place of search | Date of completion of the search | Examiner |
|---|---|---|
| The Hague | 25-03-1982 | VAN GOETHEM |

EPO Form 1503.1 06.78